# EUROPEAN PATENT SPECIFICATION

(11) **EP 0 654 021 B1**
(45) Date of publication and mention of the grant of the patent: **04.12.1996**
(21) Application number: 93918667.2
(22) Date of filing: 05.08.1993
(51) Int. Cl.: C07C 2/66

(54) **A NEW PROCESS FOR AROMATICS ALKYLATION**
VERFAHREN ZUR ALKYLIERUNG VON AROMATISCHEN VERBINDUNGEN
NOUVEAU PROCEDE D'ALKYLATION DE COMPOSES AROMATIQUES

(30) Priority: 05.08.1992 US 926093
(43) Date of publication of application: 24.05.1995
(73) Proprietor: EXXON CHEMICAL PATENTS INC., Baytown, TX 77520-2149 (US)
(72) Inventor: OU, John, Di-Yi, Houston, TX 77062 (US)
(74) Representative: Bawden, Peter Charles
(86) International application number: PCT/US93/07379
(87) International publication number: WO 94/03416

(56) References cited:
- EP-A- 0 012 514
- EP-A- 0 433 932
- US-A- 4 761 513

## Description

### BACKGROUND OF THE INVENTION

The present invention relates to methods of alkylating aromatic compounds which maximizes the life of the alkylation catalyst.

In conventional alkylation processes, the aromatic hydrocarbons and the alkylating agent are usually added in a single stage with a slight excess of aromatic to alkylating agent, e.g., in a range between about 2:1 to 20:1, to reduce the formation of the undesirable heavy products. However, lower excesses of aromatic have been favored because too much excess aromatic was believed to result in less monoalkylation product, e.g., as disclosed in U.S. Patent No. 4,400,570 to Butler, et al. However, it has also been proposed, e.g., in U.S. Patent No. 4,761,513 to STEACY, to proportionally add the alkylating agent to the aromatic hydrocarbon at the intermediate inlets of reaction zones to result in a molar ratio of aromatic hydrocarbon to alkylating agent in the range of 1:1 to about 50:1 in the feed to the reaction zones and to fulfill the quenching requirements for the process.

Solid acid catalysts typically are used to catalyze the alkylation of aromatic compounds.

One major problem that has been encountered when alkylating aromatics using solid acid catalysts is rapid deactivation of the catalyst. A cause of such deactivation appears to lie in the alkylation mechanism itself, wherein carbonium ions are formed from the alkylation agent and the acid catalyst during the first step of the alkylation reaction. In the next stage of the reaction some of the carbonium ions are added to the aromatic ring to finish the alkylation process, while other carbonium ions react with the remaining alkylation agent to produce high molecular weight compounds which, in turn, can alkylate aromatics resulting in even heavier compounds. The high molecular weight compounds are undesirable because they may deactivate the catalyst by permanently blocking the acidic sites which catalyze the alkylation reaction.

A number of methods have been tried to overcome the problem of deactivation of acid catalysts during the alkylation of aromatics. For example, others have tried: increasing or decreasing the particle size of the catalyst; multiple reaction beds for serial use after deactivation of the catalyst; recycling of the mono- or di- alkylation product back to the initial reaction mixture; and, co-feeding steam along with the aromatic and the alkylating agent.

EP-A-0 433 932 employs a new catalyst comprising an acidic mordenite zeolite having a silica/alumina molar ratio of at least 30:1 and a crystalline structure with Symmetry Index of at least 1, together with an inert silica binder, to alkylate benzene or substituted benzene. Benzene/alkylating agent ratios of 10:1 to 3:1 are taught, and the alkylating agent may be introduced to the reaction on demand until the desired degree of conversion is achieved.

EP-A-0 012 514 teaches an alkylation process performed in the liquid phase at relatively low temperatures of 100 to 300°C using a zeolite catalyst. A suitable aromatic/alkylating agent mole ratio is in the range 20:1 to 1:1, and Weight Hourly Space Velocities for benzene/propylene of 31.0/1.8, 30/24 and 15.4/1.2 are exemplified in Table XV.

### SUMMARY OF THE INVENTION

According to the present invention there is provided a method for alkylating aromatic compounds by passing a mixture of an aromatic hydrocarbon and an alkylation agent over an alkylation catalyst in a reactor under alkylating conditions sufficient to result in alkylation of the aromatic hydrocarbon and adding alkylation agent to the mixture to replenish alkylation agent depleted during alkylation of the aromatic hydrocarbon, characterized in that the molar ratio of aromatic hydrocarbon to alkylation agent is controlled at a level of at least 99:1 to reduce or minimize deactivation of the alkylation catalyst during the alkylation.

### BRIEF DESCRIPTION OF THE DRAWINGS

Figure 1 is a diagrammatic representation of a single reactor having multiple injection ports for use in injecting the alkylation agent according to the present invention.

Figure 2 is a diagrammatic representation of multiple reactors connected in series having injection ports for the alkylation agent just prior to each reactor.

Figure 3 is a chart illustrating the rate of deactivation of an acid catalyst using conventional alkylation conditions, as described in Example 1. The concentration of alkylated meta-xylene, ethylbenzene, and ortho-xylene present in the reaction product are charted as a function of bed volume of feed pumped through the reactor. The yield for all three compounds is shown to decrease steadily with time, indicating gradual catalyst deactivation.

Figure 4 is a diagrammatic representation of the experimental setup described in Example 2.

Figure 5 is a chart illustrating the relatively stable activity of an acid catalyst used in the present process, as described in Example 2. The concentration of alkylated meta-xylene, ethylbenzene, and ortho-xylene present in the product are charted as a function of bed volume of feed pumped through the reactor. The yield for all three compounds decreased more slowly with time than the decrease shown in Figure 3, indicating a slower catalyst deactivation using the present invention.

Figure 6 is a chart illustrating the concentration of alkylated ethylbenzene, meta-xylene, and ortho-xylene present in the product charted as a function of bed volumes of feed pumped through the reactor. The catalyst was deactivated faster than under the conditions of Figure 5.

### DETAILED DESCRIPTION OF THE INVENTION

The present invention provides a method for increasing the alkylation of an aromatic compound while at the same time optimizing the life of the acid catalyst used to alkylate the aromatic compound.

The method involves providing an amount of alkylating agent in the reaction mixture to result in an excess of at least 99:1 of aromatic, and replenishing spent alkylation agent at points in the reactor in order to maintain the desired excess of aromatic whilst permitting the reaction to proceed, thereby effectively prolonging the life of the catalyst. For example, the alkylating agent may be introduced intermittently, or in stages, to the aromatic compound.

For continuous operation, a single reactor equipped with multiple injection ports may be used. Alternatively, multiple reactors may be connected in series adapted so that the alkylation agent may be introduced upstream of the entrance to each reactor.
Preferably the reactor is a fixed bed reactor.

The alkylation method of the present invention is effective in minimizing or preventing deactivation or otherwise optimizing the life of a number of acid catalysts during alkylation of essentially any aromatic compound, such as substituted or unsubstituted aromatic compounds, e.g., benzene, toluene, and xylene.

Alkylating agents suitable for use in accordance with the present invention include members selected from the group consisting of olefins, paraffins, and alcohols. Examples of suitable olefins include butene and propylene; examples of suitable paraffins include propane, butane, pentane, and hexanes; examples of alcohols include isopropyl alcohol and isobutyl alcohol. Other suitable alkylating agents include halogenated paraffins, such as chloro-butane and chloro-propane.

Examples of alkylation catalysts include acid catalysts, such as zeolites, ion exchange resins, clay, silica, alumina, alumino silicates, and solid acids.

In a preferred embodiment, the invention is used to prevent deactivation of zeolites used to catalyze the alkylation of xylenes and/or alkylbenzenes.

According to the present invention, the reaction mixture to which the catalyst is exposed is comprised of an alkylating agent and an excess of aromatic, described herein as a molar ratio of alkylatable aromatic hydrocarbon and alkylating agent, of above 99:1, preferably above 200:1 or 500:1, more preferably above 900:1, and most preferably about 999:1 or above. Lower concentrations of the alkylation agent are preferred because it has been discovered that higher concentrations of the alkylation agent increase the rate at which the catalyst becomes deactivated. Preferably conditions are employed such that catalyst deactivation is reduced or minimized, e.g., to the state where activity is maintained for at least 10 times the active life of the catalyst absent the specified control.

For example, where an excess aromatic ratio of 99:1 is used without replenishment of alkylation agent during the alkylation of xylene using a zeolite catalyst, the catalyst may be deactivated after only a single day of operation. However, if an excess of aromatic of approximately 999:1 is used during the alkylation of xylene using a zeolite catalyst, it is possible for the catalyst to remain active for as long as two weeks. Other catalysts may be expected to behave similarly. In accordance with the present invention, therefore, an amount of excess aromatic may be selected according to the particular needs of the plant involved to control the rate of deactivation of the alkylation catalyst so as to minimize catalyst deactivation and otherwise optimize the life of the catalyst.

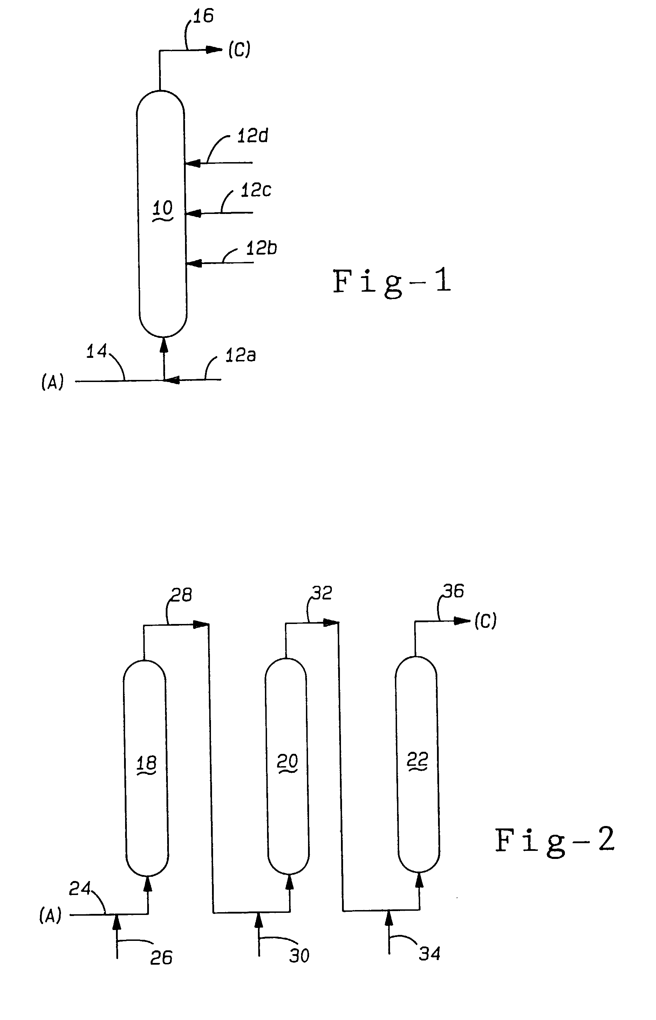

The present invention is based on the discovery that the rate of deactivation of the alkylation catalyst can be varied by controlling the molar ratio of alkylatable aromatic hydrocarbon and alkylating agent. More specifically, the rate of deactivation of the alkylation catalyst has been discovered to vary inversely to the molar ratio of alkylatable aromatic hydrocarbon and alkylating agent. In accordance with the present invention, therefore, a molar ratio of alkylatable aromatic hydrocarbon and alkylating agent is predetermined for a rate of deactivation of the alkylation catalyst that will result in a suitable length of operation before the catalyst becomes deactivated to an extent that requires regeneration. Thus, a mixture of alkylatable aromatic hydrocarbon and alkylating agent in an appropriate predetermined molar ratio of at least 99:1 is initially introduced into a reactor in which the alkylation of the aromatic is performed. As alkylation of the aromatic proceeds, the amount of alkylating agent present in the mixture is depleted in the production of alkylated aromatic hydrocarbon product. Therefore, alkylating agent is added to replenish the depleted alkylating agent in the mixture. The alkylating agent may be added to the mixture periodically or intermittently, or may be added in stages, as described in more detail herein below with respect to the operation of the present invention as shown in Figures 1 and 2. Although alkylation of the aromatic may be permitted to proceed or progress to an extent where essentially all of the alkylating agent is depleted before adding additional alkylating agent to replenish the depleted alkylating agent in the mixture, it is preferred to add the alkylating agent in a manner that maintains a sufficient amount of alkylating agent to be effective in maintaining a substantially continuous alkylation of the aromatic. Preferably, the addition of alkylating agent results in a molar ratio of alkylatable aromatic hydrocarbon and alkylating agent similar to their molar ratio in the mixture that was initially introduced into the reactor. The key is that at no time during the alkylation process is the concentration of alkylation agent allowed to reach levels which, although suitable for alklation, cause substantial catalyst deactivation. Thus the starting concentration of alkylation agent is below this deactivating level, and it is never relinquished any stage to such a deactivating level. This means that the total amount of alkylation agent which is required for complete conversion of the aromatic hydrocarbon is not present initially, but is only added as the reaction proceeds, as the alkylation agent which is initially present becomes used up.

Schematics illustrating the operation of the present invention are shown in Figures 1 and 2 wherein the concentration of the alkylation agent that should be present in the reaction mixture is assumed to be less than "C". In Figure 1, the feed (A) enters single reactor 10 through conduit or line 14, which reactor is filled with a bed of acid catalyst, and is provided with multiple stages or injection ports 12a-d. An injection port 12a is provided in line 14 before the product feed enters the reactor in order to inject the alkylation agent into the reaction mixture in an amount that will result in a concentration of Cₘ (or less) of the alkylation agent in the reaction mixture (where "Cₘ" designates the maximum desirable concentration of alkylation agent which is present). In addition to injection ports 12a-d, reactor 10 should have means for supplying suitable temperatures and pressures (not shown) for the alkylation of aromatics, e.g., temperatures up to 500°C (932° F), and pressures of up to 6.89 MPag (1000 psig). Preferably, alkylation of aromatics in accordance with the present invention is performed under alkylation conditions including a temperature of less than 500°C, and more preferably in a range of 10°C to 300°C, and/or a pressure within a range from sub-atmospheric to 6.89 MPag (1000 psig), and/or at a Liquid Hourly Space Velocity (LHSV) within a range of 0.1 to 100. However, alkylation conditions, such as temperature may be varied to be suitable for particular alkylation reactions.

As the reaction mixture is pumped through reactor 10, and as the alkylation agent reacts with the aromatic feed, the reaction mixture passes a second injection port 12b though which alkylation agent is injected in an amount that will result in a concentration of Cₘ or less of the alkylation agent in the reaction mixture. Additional injections of the alkylation agent occur as the reaction mixture is pumped past injection ports 12c and 12d. At each injection port 12 a-d, the amount of alkylation agent that is injected should be carefully set or monitored so that the concentration of alkylation agent in the reaction mixture is controlled to as not to exceed approximately Cₘ. Of course, reactor 10 may be supplied with any number of injection ports 12a-d. Once the reaction mixture is pumped through the reactor 10, the product (C) passes out of reactor 10 through line 16.

Figure 2 is a diagrammatic representation of an alternate means for intermittently injecting an alkylation agent according to the present invention, wherein reactors 18, 20, and 22 are provided in series. The product feed (A) enters the first reactor 18 through conduit or line 24 that is provided with an injection port 26 for injecting the alkylation agent into the product feed before the product feed enters the reactor 18.

The effluent leaves the first reactor 18 via the conduit or line 28, which also serves to feed the effluent from reactor 18 into the second reactor 20. Conduit 28 is provided with an injection port 30 which is positioned, similar to injection port 26, upstream of reactor 20 in conduit 28.

The effluent exits reactor 20 via line 32; the alkylation agent is added to a concentration of Cₘ or less via injection port 34 before the product stream enters reactor 22; and, the effluent (C) exits reactor 22 via line 36. Of course, one could add as many or as few reactors in series as required for specific needs.

### Example 1 (Comparative)

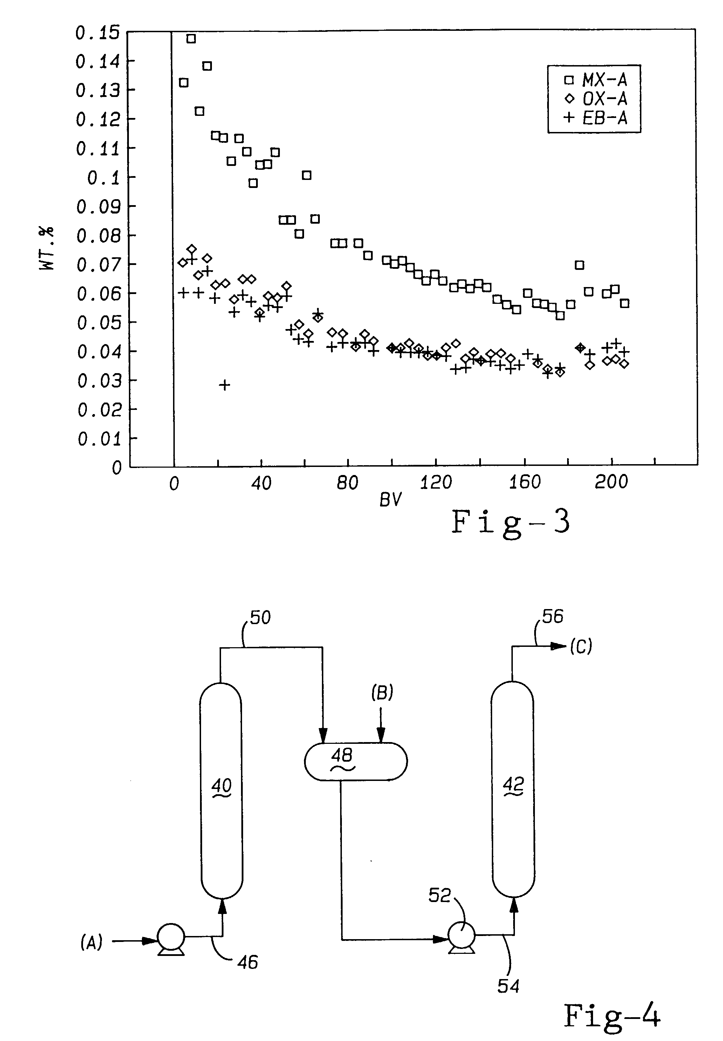

Example 1 illustrates the catalyst deactivation problem that usually occurs in a conventional, single stage, fixed-bed alkylation process. In this alkylation process, meta-xylene (MX), or ortho-xylene (OX) and ethylbenzene (EB) were selectively alkylated in a medium of para-xylene (PX) using isobutylene as the alkylation agent. Due to steric hindrance, isobutylene has a higher alkylation selectivity toward MX, OX, and EB than to PX. The feed stream contained 99.746% PX, 0.137% MX, 0.067% EB, and 0.050% OX. The catalyst, a hydrogen-form Y zeolite (LZY-84) provided by UOP Inc., was loaded into a 1.27 x 8.89 cm (1/2" x 3-1/2") stainless steel reactor. A mixture of 99.70% feed and 0.30% isobutylene was pumped through the reactor at 1 liquid hourly space velocity (LHSV), 344.75 KPag (50 psig), and 22°C (71.6°F). Reaction product was collected periodically and analyzed for xylene and alkylated xylene distributions. Figure 3 shows the concentration of alkylated MX, EB and OX (MX-A, EB-A, OX-A) in product as a function of bed volumes (BV) of feed pumped through the reactor, and that the yield for all three compounds decreased with time indicating a gradual catalyst deactivation.

### Example 2 (Invention)

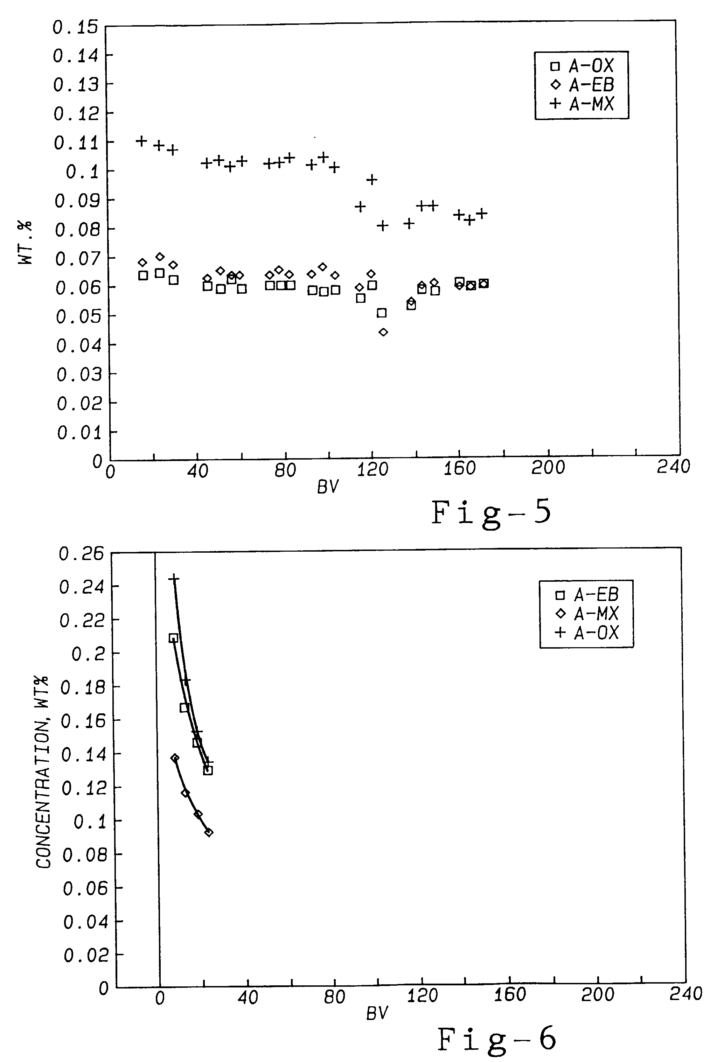

This example illustrates the advantage of the present invention with respect to catalyst stability and yields. Figure 4 is a diagrammatic representation of the set up used in this example, wherein two 1.27 X 4.445 cm (1/2" x 1-3/4") reactors 40, 42 were arranged serially, with the volume of each of the two reactors being exactly half of the reactor volume in Example 1. The same xylene stream used in Example 1 was used for feed. A mixture (A) of 99.90% feed and 0.10% isobutylene was pumped via line 46 into the first reactor 40 at 2 LHSV, 344.75 kPag (50 psig), and 22°C (71.6°F). The effluent from the first reactor 40 then was pumped to storage tank 48 via line 50. In storage tank 48, 0.06% isobutylene (B) was added to the product stream. The product stream then was pumped from storage tank 48 by pump 52 through line 54 and into the second reactor 42, and maintained at the same conditions as in the first reactor 40, exiting as final product (C) through line 56. Although the individual flow rate for each reactor was 2 LHSV, the overall flow rate for the two reactors in series was 1 LHSV. The concentration of alkylated MX, OX, and EB (A-MX, A-OX, A-EB) is plotted in Figure 5, which shows that the present invention provides a more efficient use of isobutylene (0.16% vs. 0.30%) with similar initial yields to those of Example 1, in addition to providing greater catalyst stability, as demonstrated by the relatively stable yields for all three compounds.

### Example 3 (Comparative)

This example illustrates the alkylation results at a hydrocarbon to isobutylene ratio of 99:1, with no staging of the reaction, wherein the hydrocarbon contained 98.98% para-xylene, 0.49% meta-xylene, 0.33% ethylbenzene, and 0.20% ortho-xylene. A mixture of 99.00% hydrocarbon and 1.00% isobutylene was pumped through a 1.27 X 4.445 cm (1/2" x 1-3/4") stainless steel reactor packed with LZY-84 catalyst at 1 LHSV, 344.75 kPag (50 psig), and 22°C. Reaction product was collected periodically and analyzed for xylene and alkylated xylene distribution. Figure 6 shows the concentration of alkylated ethylbenzene, meta-xylene, and ortho-xylene in the reaction product as a function of the bed volumes of feed pumped through the reactor and that the catalyst was deactivated faster than under the conditions of Example 2.

## Claims

1. A method for alkylating aromatic compounds by passing a mixture of an aromatic hydrocarbon and an alkylation agent over an alkylatio catalyst in a reactor under alkylating conditions sufficient to result i alkylation of the aromatic hydrocarbon and adding alkylation agent t the mixture as alkylation proceeds, to replenish alkylation age depleted during alkylation of the aromatic hydrocarbon, characterize in that the molar ratio of aromatic hydrocarbon to alkylation agent controlled to a level of at least 99:1 to reduce deactivation of the alkylation catalyst during the alkylation.

2. The method of Claim 1 wherein the alkylation agent is added to the mixture at sequential zones of a single flow-through reactor.

3. The method of Claim 1 wherein the alkylation agent is added to the mixture at sequential reactors of a series of flow-connected reactors.

4. The method of any preceding claim wherein the molar ratio controlled to a level of at least 200:1, preferably at least 500:1, more preferably at least 900:1.

5. The method of Claim 4 wherein the molar ratio is controlled to a level of at least 999:1.

6. The method of any preceding claim wherein the alkylating conditions comprise a temperature from 10°C to 300°C and/or a pressure from sub-atmospheric to 6.89 MPag (1000 psig) and/or a LHSV between 0.1 and 100.

7. The method of any preceding claim wherein the reactor is a fixed bed reactor.

8. The method of any preceding claim wherein the aroma hydrocarbon comprises xylene.

9. The method of any preceding claim wherein the catalyst is an acid catalyst.

10. The method of Claim 9 wherein the catalyst is a zeolite.

## Patentansprüche

1. Verfahren zum Alkylieren von aromatischen Verbindungen, bei dem eine Mischung aus einem aromatischen Kohlenwasserstoff und einem Alkylierungsmittel über einen Alkylierungskatalysator in einem Reaktor unter Alkylierungsbedingungen geleitet wird, die ausreichen, um zur Alkylierung des aromatischen Kohlenwasserstoffs zu führen, und der Mischung im Verlauf der Alkylierung Alkylierungsmittel zugesetzt wird, um das Alkylierungsmittel zu ersetzen, das während der Alkylierung des aromatischen Kohlenwasserstoffs verbraucht worden ist, dadurch gekennzeichnet, daß das Molverhältnis von aromatischem Kohlenwasserstoff zu Alkylierungsmittel auf eine Höhe von mindestens 99:1 geregelt wird, um die Desaktivierung des Alkylierungskatalysators während der Alkylierung zu verringern.

2. Verfahren nach Anspruch 1, bei dem das Alkylierungsmittel der Mischung in aufeinanderfolgenden Zonen eines einzigen Durchflußreaktors zugesetzt wird.

3. Verfahren nach Anspruch 1, bei dem das Alkylierungsmittel der Mischung in aufeinanderfolgenden Reaktoren einer Reihe von durchflußverbundenen Reaktoren zugesetzt wird.

4. Verfahren nach einem der vorhergehenden Ansprüche, bei dem das Molverhältnis auf eine Höhe von mindestens 200:1, vorzugsweise mindestens 500:1, insbesondere mindestens 900:1 geregelt wird.

5. Verfahren nach Anspruch 4, bei dem das Molverhältnis auf eine Höhe von mindestens 999:1 geregelt wird.

6. Verfahren nach einem der vorhergehenden Ansprüche, bei dem die Alkylierungsbedingungen eine Temperatur von 10°C bis 300°C und/oder einen Druck von subatmosphärischem bis 6,89 MPa Überdruck (1000 psig) und/oder ein LHSV zwischen 0,1 und 100 einschließen.

7. Verfahren nach einem der vorhergehenden Ansprüche, bei dem der Reaktor ein Festbettreaktor ist.

8. Verfahren nach einem der vorhergehenden Ansprüche, bei dem der aromatische Kohlenwasserstoff xylol umfaßt.

9. Verfahren nach einem der vorhergehenden Ansprüche, bei dem der Katalysator ein saurer Katalysator ist.

10. Verfahren nach Anspruch 9, bei dem der Katalysator ein Zeolith ist.

## Revendications

1. Procédé pour alkyler des composés aromatiques par passage d'un mélange d'un hydrocarbure aromatique et d'un agent d'alkylation sur un catalyseur d'alkylation dans un réacteur dans des conditions d'alkylation suffisantes pour qu'il en résulte l'alkylation de l'hydrocarbure aromatique, et par addition d'agent d'alkylation au mélange lorsque l'alkylation s'effectue, pour renouveler l'agent d'alkylation épuisé au cours de l'alkylation de l'hydrocarbure aromatique, caractérisé en ce que le rapport molaire de l'hydrocarbure aromatique à l'agent d'alkylation est ajusté à une valeur d'au moins 99:1 pour réduire la désactivation du catalyseur d'alkylation au cours de l'alkylation.

2. Procédé suivant la revendication 1, dans lequel l'agent d'alkylation est ajouté au mélange au niveau de zones successives d'un seul réacteur à circulation.

3. Procédé suivant la revendication 1, dans lequel l'agent d'alkylation est ajouté au mélange au niveau de réacteurs successifs d'une série de réacteurs connectés par écoulement.

4. Procédé suivant l'une quelconque des revendications précédentes, dans lequel le rapport molaire est ajusté à une valeur d'au moins 200:1, avantageusement d'au moins 500:1, de préférence d'au moins 900:1.

5. Procédé suivant la revendication 4, dans lequel le rapport molaire est ajusté à une valeur d'au moins 999:1.

6. Procédé suivant l'une quelconque des revendications précédentes, dans lequel les conditions d'alkylation comprennent une température de 10°C à 300°C et/ou une pression allant d'une valeur inférieure à la pression atmosphérique à 6,89 MPa au manomètre (1000 psig) et/ou une VSHL comprise dans l'intervalle de 0,1 à 100.

7. Procédé suivant l'une quelconque des revendications précédentes, dans lequel le réacteur est un réacteur à lit fixe.

8. Procédé suivant l'une quelconque des revendications précédentes, dans lequel l'hydrocarbure aromatique comprend le xylène.

9. Procédé suivant l'une quelconque des revendications précédentes, dans lequel le catalyseur est un catalyseur acide.

10. Procédé suivant la revendication 9, dans lequel le catalyseur est une zéolite.
